(19) Europäisches Patentamt
European Patent Office
Office européen des brevets

(11) **EP 3 315 134 B1**

(12) **EUROPEAN PATENT SPECIFICATION**

(45) Date of publication and mention
of the grant of the patent:
**02.09.2020   Bulletin 2020/36**

(51) Int Cl.:
*A61K 35/34* *(2015.01)*      *A61P 35/00* *(2006.01)*

(21) Application number: **17198789.4**

(22) Date of filing: **27.10.2017**

(54) **COMPOSITION FOR USE IN TUMOR GROWTH INHIBITION AND PREVENTION OF CANCER CELL METASTASIS BY IMPLANTATION COMPRISING MYOBLASTS**

ZUSAMMENSETZUNG ZUR VERWENDUNG IN DER TUMORWACHSTUMSHEMMUNG UND PRÄVENTION VON KREBSZELLMETASTASIERUNG DURCH IMPLANTATION ENTHÄLTEND MYOBLASTEN

COMPOSITION POUR L'ULITILISATION DANS L'INHIBITION DE LA CROISSANCE TUMORALE ET LA PRÉVENTION DE LA MÉTASTASE DES CELLULES CANCÉREUSES PAR IMPLANTATION COMPRENANT DES MYOBLASTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **27.10.2016   PCT/IB2016/056459**

(43) Date of publication of application:
**02.05.2018   Bulletin 2018/18**

(73) Proprietor: **Law, Peter K.**
**Richmond Hill, Ontario L4B 2L1 (CA)**

(72) Inventor: **Law, Peter K.**
**Richmond Hill, Ontario L4B 2L1 (CA)**

(74) Representative: **Hinkelmann, Klaus**
**Patentanwaltskanzlei Hinkelmann**
**Lyonel-Feininger-Strasse 28**
**80807 München (DE)**
(56) References cited:
**EP-A1- 2 837 683     WO-A1-96/18303**

• **MELINE NOGUEIRA LUCENA STÖLTING ET AL: "Myoblasts Inhibit Prostate Cancer Growth by Paracrine Secretion of Tumor Necrosis Factor-[alpha]", JOURNAL OF UROLOGY., vol. 189, no. 5, 1 May 2013 (2013-05-01), pages 1952-1959, XP055456395, BALTIMORE, MD, US ISSN: 0022-5347, DOI: 10.1016/j.juro.2012.10.071**
• **OZAWA C R ET AL: "A novel Means Drug Delivery: Myoblast-Mediated Gene Therapy and Regulatable Retoviral Vectors", ANNUAL REVIEW OF PHARMACOLOGY AND TOXICOLOGY, ANNUAL REVIEW INC., PALO ALTO, CA, US, vol. 40, 1 January 2000 (2000-01-01), pages 295-317, XP002985273, ISSN: 0362-1642, DOI: 10.1146/ANNUREV.PHARMTOX.40.1.295**

**Description**

The present invention is related to a use of a composition comprising myoblasts for inhibiting growth of a cancer tumor in a host and/or preventing cancer cells from metastasis in a host

The National Cancer Institute describes cancer on its homepage as follows. Cancer is the name given to a collection of related diseases. In all types of cancer, some of the body's cells begin to divide without stopping and spread into surrounding tissues. Cancer can start almost anywhere in the human body, which is made up of trillions of cells. Normally, human cells grow and divide to form new cells as the body needs them. When cells grow old or become damaged, they die, and new cells take their place. When cancer develops, however, this orderly process breaks down. As cells become more and more abnormal, old or damaged cells survive when they should die, and new cells form when they are not needed. These extra cells can divide without stopping and may form growths called tumors.

Many cancers form solid tumors, which are masses of tissue. Cancers of the blood, such as leukemias, generally do not form solid tumors. Cancerous tumors are malignant, which means they can spread into, or invade, nearby tissues. In addition, as these tumors grow, some cancer cells can break off and travel to distant places in the body through the blood or the lymph system and form new tumors far from the original tumor.

Unlike malignant tumors, benign tumors do not spread into, or invade, nearby tissues. Benign tumors can sometimes be quite large, however. When removed, they usually don't grow back, whereas malignant tumors sometimes do. Unlike most benign tumors elsewhere in the body, benign brain tumors can be life threatening.

Cancer is a genetic disease-that is, it is caused by changes to genes that control the way our cells function, especially how they grow and divide. Genetic changes that cause cancer can be inherited from our parents. They can also arise during a person's lifetime as a result of errors that occur as cells divide or because of damage to DNA caused by certain environmental exposures. Cancer-causing environmental exposures include substances, such as the chemicals in tobacco smoke, and radiation, such as ultraviolet rays from the sun. Each person's cancer has a unique combination of genetic changes. As the cancer continues to grow, additional changes will occur. Even within the same tumor, different cells may have different genetic changes. In general, cancer cells have more genetic changes, such as mutations in DNA, than normal cells. Some of these changes may have nothing to do with the cancer; they may be the result of the cancer, rather than its cause.

In metastasis, cancer cells break away from where they first formed (primary cancer), travel through the blood or lymph system, and form new tumors (metastatic tumors) in other parts of the body. The metastatic tumor is the same type of cancer as the primary tumor. A cancer that has spread from the place where it first started to another place in the body is called metastatic cancer. The process by which cancer cells spread to other parts of the body is called metastasis.

Metastatic cancer has the same name and the same type of cancer cells as the original, or primary, cancer. For example, breast cancer that spreads to and forms a metastatic tumor in the lung is metastatic breast cancer, not lung cancer. Under a microscope, metastatic cancer cells generally look the same as cells of the original cancer. Moreover, metastatic cancer cells and cells of the original cancer usually have some molecular features in common, such as the presence of specific chromosome changes. Treatment may help prolong the lives of some people with metastatic cancer. In general, though, the primary goal of treatments for metastatic cancer is to control the growth of the cancer or to relieve symptoms caused by it. Metastatic tumors can cause severe damage to how the body functions, and most people who die of cancer die of metastatic disease [www.cancer.gov].

Up to now one of the most common treatment strategies for cancer is the use of one or more anti-cancer drugs, also known as chemotherapeutic agents as regimen of a standardized chemotherapeutic regimen. Chemotherapy may be given with a curative intent or it may aim to prolong life or to reduce symptoms (palliative chemotherapy). Chemotherapy is one of the major categories of medical oncology.

By common usage, the term chemotherapy has come to connote the use of rather nonspecific intracellular poisons, especially related to inhibiting the process of cell division known as mitosis, and generally excludes agents that more selectively block extracellular growth signals (i.e. blockers of signal transduction). Importantly, the use of drugs constitutes "systemic therapy" for cancer in that they are introduced into the blood stream and are therefore in principle able to address cancer at any anatomic location in the body. Systemic therapy is often used in conjunction with other modalities that constitute "local therapy" (i.e. treatments whose efficacy is confined to the anatomic area where they are applied) for cancer such as radiation therapy, surgery, and/or hyperthermia therapy.

Traditional chemotherapeutic agents are cytotoxic by means of interfering with cell division (mitosis) but cancer cells vary widely in their susceptibility to these agents. To a large extent, chemotherapy can be thought of as a way to damage or stress cells, which may then lead to cell death if apoptosis is initiated. Many of the side effects of chemotherapy can be traced to damage to normal cells that divide rapidly and are thus sensitive to anti-mitotic drugs: cells in the bone marrow, digestive tract, and hair follicles. This results in the most common side-effects of chemotherapy: myelosuppression (decreased production of blood cells, hence also immunosuppression), mucositis (inflammation of the lining of the digestive tract), and alopecia (hair loss) [wikipedia]. It would thus be desirable, if a more targeted cancer treatment could be provided that would not damage normal cells and have fewer side effects.

Evolution has muscles protecting all vertebrates against predation including diseases. From the divergence of placental and marsupial unto the present day, there existed over 160 million years of evolution during which the mammalian body was exposed to numerous carcinogenic attacks [Zhe-Xi Luo et al., Nature 2011, 476: 442-445]. Despite the involvement of most differentiated cell types in the human body, myogenic cells appear to be relatively spared of carcinogenicity. Reports of malignant tumor metastases in cardiac, skeletal and smooth muscles have been rare. This led the inventor to believe that the myogenic cells might have developed in the course of evolution certain mechanism(s) to defy carcinogenic insults.

The use of myoblasts for treating disease conditions is already known.

The publication EP 1 407 788 A2 discloses a method of treating mammalian disease conditions that are hereditary, degenerative, debilitating, fatal or undesirable, comprising the steps of: culturing a supply of myogenic cells that are normal, genetically transduced or phenotypically converted, the myogenic cells comprising myoblasts, myotubes, young muscle fibers and converted cell types; administering a therapeutically effective dosage of an immunosuppressant to the host; and thereafter selecting and administering from said supply a therapeutically effective dose of myogenic cells or cells converted from myogenic cells to the host, whereby tissue/organ size, shape and/or function are improved and/or the disease condition(s) are prevented, alleviated or annihilated.

The publication EP 2 837 683 A1 discloses a composition comprising a supply quantum of cultured genetically normal myoblasts supplying a complete genome of the host species for use in treating and/or preventing a genetic disease in a host, wherein it comprises the myoblasts in host serum. In particular, EP 2 837 683 A1 is concerned with muscle regeneration by implanting myoblasts of the host into hereditary degenerating diseases. There is no mention of cancer and cancer treatment.

Studies on co-cultures of myoblasts and tumor cells are also already known.

The publication Stölting, MNL et al., J Urol 2013, 189:1952-1959 reports that myoblasts restricted prostate cancer growth and metastasis by paracrine TNF-$\alpha$ secretion. An increase up to 25 fold of TNF-$\alpha$ mRNA basal level was demonstrated when myoblasts were co-cultured with tumor cells. Co-culture experiments revealed induction of cell cycle arrest, tumor death by apoptosis and increased myoblast differentiation. This effect was largely blocked by TNF-$\alpha$ inhibition. The same outcome was noted in nude mice, in which co-injected human myoblasts inhibited the tumor growth and lymph node metastasis of all prostate cancer cell lines evaluated. The authors conclude that myoblasts are a promising cell source for muscle reconstruction even in the proximity of cancer which illustrates that the authors were pursuing muscle reconstruction rather than teaching or suggesting cancer treatment.

The publication WO 96/18303 A1 describes that co-culture of myoblasts and melanoma cancer cells in myoblast fusion medium results in cell death of the melanoma cancer cells, suggesting the potential use of myoblasts in the treatment of cancer. In an experiment shown in its Fig. 18, normal myoblasts and malignant melanoma CRL6322 cells were co-cultured for 4 days in myoblast growth medium followed by co-culturing in myoblast fusion medium (low serum content of 2 %). After 19 days culturing in the myoblast fusion medium, melanoma cells became spherical and detached (dying cells), whereas myoblasts began to fuse forming myotubes. This effect of dying melanoma cells and surviving fusion myoblasts was more pronounced when myoblasts and melanoma cells were seeded initially at 3:1 concentration ratio in comparison to 1:1 concentration ratio.

The publication Ozawa, C.R. et al. "A novel means drug delivery: myoblast-mediated gene therapy and regulatable retroviral vectors". Annual Review of Pharmacology and Toxicology. Annual Review INC., vol. 40, 1 January 2000. pages 295-317, gives an overview of myoblast-mediated gene therapy and suggests the potential use of genetically engineered myoblasts expressing anti-angiogenic proteins in the inhibiting of tumor metastasis.

In view of this situation, an object underlying the present invention is to provide a potent and effective way of treating cancer in a host. Ideally, a composition should be provided that is at the same time safe to apply and comparably gentle in its side effects.

In accordance with the present invention, this object is achieved by composition for use in inhibiting growth of a cancer tumor in a host and/or preventing cancer cells from metastasis in a host with the features of the independent claims. Preferred embodiments of the invention are detailed in the dependent claims.

The invention is thus directed to a composition for use in inhibiting growth of a cancer tumor in a host and/or preventing cancer cells from metastasis in a host, wherein the composition is implanted into and around the tumor and/or the cancer cells such that the amount of host serum is reduced upon the implantation and the host serum reduction terminates myoblast proliferation and induces TNF-alpha release and myoblast fusion, wherein the composition comprises a quantum of genetically normal non-host myoblasts and host serum and is adapted to induce myoblast proliferation before being implanted and to induce myoblast fusion upon implantation in that a host serum level in the composition is above a content of 15 % by weight in the composition, and wherein the composition is implanted in at least one dosage containing 75 million to 250 million myoblasts per millilitre of the composition, and wherein the cancer belongs to the group consisting of melanoma cancer, prostate cancer, one or more breast cancers, gastrointestinal cancer, lung cancer, Ehrlich ascites cancer and liver cancer.

The concentration of the genetically normal myoblasts in numbers per millilitre of composition and/or the amount of host serum is chosen as such that the myoblasts proliferate in the inventive composition. Thus, the composition may have the same properties as a growth medium, in particular before implantation.

The concentration of myoblasts in numbers per milliliter of composition and/or the amount of host serum in the inventive composition is as indicated above. Other components that may be used in the inventive composition are human albumin and/or saline solution. However, preferably the inventive composition comprises myoblasts and host serum only, in particular non-host myoblasts and host serum. Accordingly, serum and myoblasts come preferably from different persons. In any way, the inventive composition is adapted to induce myoblast proliferation.

In the present invention, the host serum level in the composition is above 15% by weight in the composition. More preferably, the host serum level is of from 20% to 100%. "Level" hereby refers to the content by weight in the composition.

In a preferred embodiment of the composition for use in inhibiting growth of a cancer tumor in a host and/or preventing cancer cells from metastasis in a host, the host serum is clotted serum.

In another preferred embodiment of the composition for use in inhibiting growth of a cancer tumor in a host and/or preventing cancer cells from metastasis in a host, the myoblasts were previously isolated, purified and cultured.

In another preferred embodiment of the composition for use in inhibiting growth of a cancer tumor in a host and/or preventing cancer cells from metastasis in a host, the genetically normal myoblasts are human myoblasts.

In another preferred embodiment of the composition for use in inhibiting growth of a cancer tumor in a host and/or preventing cancer cells from metastasis in a host, the myoblasts are allogeneic.

The invention thus allows the use of a composition according to the invention for inhibiting growth of a cancer tumor in a host and/or preventing cancer cells from metastasis in a host, wherein the inventive composition is implanted into and around the tumor and/or the cancer cells such that the amount of host serum is reduced upon the implantation and the host serum reduction terminates myoblast proliferation and induces TNF-alpha release and myoblast fusion.

The inventor has surprisingly found that co-cultures of normal human myoblasts and malignant melanoma (CRL6322) cells in fusion medium promote cancer cell death. Initially it was established that melanoma cells, without myoblasts, survived and proliferated better for 14 days in the myoblast culture medium than in the cancer cell growth medium. At 9 days after co-culture of myoblasts and melanoma cells (2:1 concentration ratio) in myoblast culture medium, both cell types underwent rigorous mitosis without any sign of exhaustion. At 14 days in culture, myoblasts dominated the culture with melanoma cells still surviving. The myoblasts did not appear to have exerted any ill effect onto the cancer cells.

The investigation was taken further by the inventor by adding a fusion medium to induce fusion of myoblasts to form myotubes. After 5 days in the myoblast fusion medium, many melanoma cells had become spherical, apoptotic and detached from the collagen surface. Myoblasts remained healthy and were beginning to fuse. At 10 days in myoblast fusion medium, numerous dead melanoma cells were floating on the culture medium surface. Myoblasts and myotubes were proliferating vigorously on the collagen. At high magnification, only a few were observed detached and dying. At 19 days in myoblast fusion media, proliferating myoblasts retained their spindle shape and aligned with each other. Melanoma cells had become spherical and detached. Significantly more myotubes were observed after 19 days in the myoblast fusion medium when myoblasts and melanoma cells were seeded initially at 3:1 concentration ratio. Whereas the myoblasts undergoing cell fusion and the newly-formed myotubes appeared to have induced cancer cell apoptosis, they were not able to have completely extinguished the cancer cells in culture.

An explanation was given by the inventor to be as follows. The switching from culture medium to fusion medium constituted a condition of serum restriction because the fusion medium contained only one-fifth of the serum as in the culture medium. Serum restriction terminated the mitotic cycle of the myoblasts, and initiated the developmental process of natural cell fusion towards myotube formation. The inventor envisioned that myoblast fusion was associated with membrane breakage with significant amount of TNF-alpha (also TNF-$\alpha$) being released. Furthermore, myoblast fusion resulting in myotube formation and development quickly deprived the melanoma cells of oxygen, nutrients and L-glutamate within the confined microenvironment of the tumor capsule. These two mechanisms were considered to be responsible for the melanoma cell death in the co-culture.

TNF-$\alpha$ is an endogenous muscle factor promoting myogenesis through activation of the p38a and Pax7 pathway. It was found that TNF-$\alpha$ mRNA basal level in C2C12 myoblasts had been shown up-regulated 273% by serum restriction [Li, Y.P. and Robert J. Schwartz, The FASEB Journal express article 10.1096/fj.00-0632fje. published online April 6, 2001].

The inventor has surprisingly found that the implantation of the inventive composition into and around a tumor and/or cancer cells induces the condition equal to serum restriction conditions, as the amount of host serum is reduced upon the implantation. The reduction in host serum thus terminates the myoblast proliferation in the inventive composition and induces TNF-alpha release and myoblast fusion. As a result the proliferation of cancer cells was found to be inhibited. Tumor volume was found to be reduced and cancer cells were found to undergo cell apoptosis.

The inventor explains this surprising finding by four mechanisms to be responsible for inhibition of cancer cell proliferation, tumor volume reduction and cancer cell apoptosis. For the first time, the following four mechanisms were revealed:

a) the tumor necrosis factor -α (TNF-α, TNF-alpha) is released following cell membrane breakage in the processes of myoblast mitosis and myoblast fusion kills cancer cells;
b) the dividing myoblasts and newly developed myotubes compete successfully and take away most if not all of the nutrients, L-glutamine and oxygen inside the tumor from the cancer cells;
c) in case allogeneic myoblasts are used in the inventive composition, the injection trauma of allogeneic myoblasts mounts local inflammatory and immunologic attacks on both myoblasts and cancer cells.
d) myoblasts wrap around cancer cells and thus prevent them from metastasis and continue to exert detrimental effects on them.

In a preferred embodiment the inventive composition is implanted in at least one dosage of from 75 million to 250 million genetically normal myoblasts per millilitre of composition. More preferably, the at least one dosage is of up to 100 million genetically normal myoblasts per millilitre of composition. In applying single dosages of up to 100 million myoblasts per millilitre of composition sticking of myoblasts to the syringe and blocking of the needle can advantageously be avoided.

Accordingly, a preferred composition of the present invention contains 75 million to 250 million myoblasts per millilitre of the composition.

In a preferred embodiment, the cancer tumor is a solid tumor.

The inventor has found that the inventive composition is advantageous if according to the present invention the cancer belongs to a group consisting of melanoma cancer, prostate cancer, one or more breast cancers, gastrointestinal cancer, lung cancer, Ehrlich ascites cancer and liver cancer.

In a more preferred embodiment the cancer is melanoma cancer.

In another more preferred embodiment the cancer is prostate cancer.

In another more preferred embodiment the cancer belongs to one or more breast cancers.

In another more preferred embodiment the cancer is gastrointestinal cancer.

In another more preferred embodiment the cancer is lung cancer.

In another more preferred embodiment the cancer is Ehrlich ascites cancer.

In another more preferred embodiment the cancer is liver cancer.

In particular, it is preferred that in case the growth of a cancer tumor is to be inhibited by the use of the inventive composition that the cancer belongs to the group consisting of gastrointestinal cancer, lung cancer, Ehrlich ascites cancer and liver cancer.

According to a further embodiment, the host is not pre-treated with an immunosuppressant. In this way, if allogeneic myoblasts are used, advantageously the allogeneic myoblast's MHC-class 1 surface thus triggers local attack on both myoblasts and cancer cells.

Herein also a method for determining a composition according to the invention practiced on non-human animal models is described but not claimed, wherein the method comprises the steps of injecting cancer cells subcutaneously into a left and a right side of a back of a non-human animal model so as to induce the growth of a left tumor and a right tumor, injecting a pre-determined volume of genetically normal myoblasts in host serum into the grown right tumor and injecting the same volume of saline solution into the grown left tumor, and determining the tumor inhibitory factor by weight and/or by volume according to the following formulas (I) and/or (II) after a pre-determined period of time from the injections:

$$TIF = \frac{(mean\ left\ tumor\ weight - mean\ right\ tumor\ weight)}{mean\ left\ tumor\ weight\ x\ 100\%} \qquad (I)$$

$$TIF = \frac{(mean\ left\ tumor\ volume - mean\ right\ tumor\ volume)}{mean\ left\ tumor\ volume\ x\ 100\%} \qquad (II)$$

The inventor has surprisingly found that the weight and/or volume of a cancer tumor are reduced by the injection of genetically normal myoblasts (right tumor) as compared to a control tumor (left tumor) of the same type, size and age. This reduction is given by the tumor inhibitory factor according to the formulas (I) and/or (II), respectively. For example, a tumor inhibitory factor of 20% could be found for nude mice after 20 days.

In a preferred embodiment of the non-claimed method a dose of from 10 to 12 million genetically normal myoblasts per millilitre of composition is injected into the right tumor.

In another preferred embodiment of the non-claimed method, the myoblasts are human myoblasts.

Generally, the non-claimed_method allows determining the inventive composition for any kind of cancer tumor and/or cancer cells. The inventor, however, has found that the method is especially advantageous if the cancer cells injected to grow a tumor on the left and the right side of the back of the non-human animal model belong to the group consisting of melanoma cancer cells, prostate cancer cells, lung cancer cells, liver cancer cells, gastrointestinal cancer cells, Ehrlich ascites cancer cells and one or more breast cancers cells.

In a preferred embodiment the injected cancer cells are lung cancer cells.

In another preferred embodiment the injected cancer cells are melanoma cancer cells.

In another preferred embodiment the injected cancer cells are prostate cancer cells.

In another preferred embodiment the injected cancer cells are liver cancer cells.

In another preferred embodiment the injected cancer cells are gastrointestinal cancer cells.

In another preferred embodiment the injected cancer cells are Ehrlich ascites cancer cells.

In another preferred embodiment the injected cancer cells are cells of one or more breast cancers.

In another preferred embodiment the injected cancer cells induce the growth of a left and a right solid tumor.

The invention has numerous advantages. By implantation of the inventive composition tumor growth can be inhibited, cancer cell apoptosis can be induced and cancer cells can be prevented from metastasis. The implantation of the inventive composition thus allows not only treating cancer effectively, but also targeted. The implantation of the inventive composition is furthermore safe showing no serious side effects. Side effects associated with cancer treatments such as chemotherapy can be avoided.

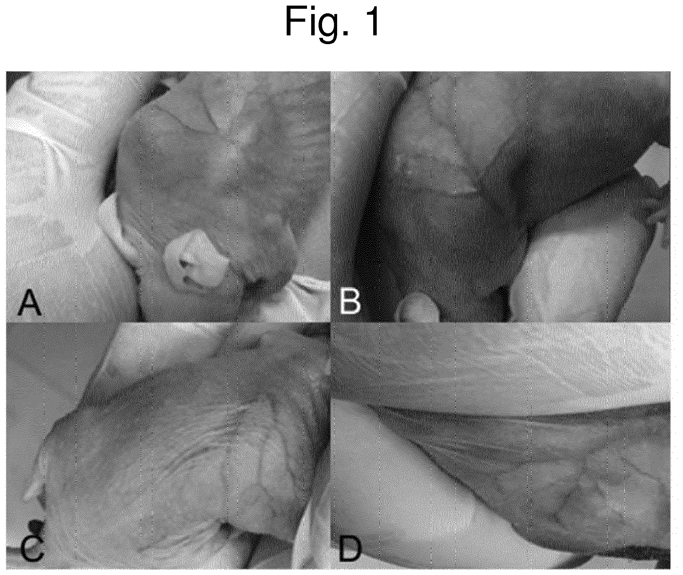

In the following, the present invention will be described in more detail by means of non limiting examples. Reference is made therein to Figures 1 to 14.

Fig. 1. Myoblasts were injected after the tumors had developed capillary network (A-D).

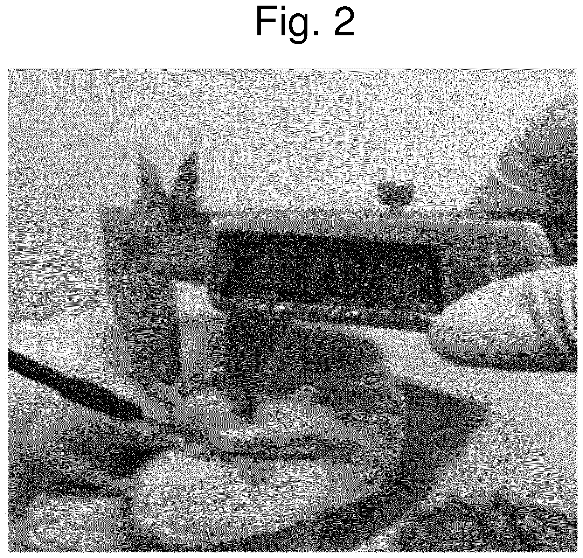

Fig. 2. A caliper was used to measure the length and width of tumors.

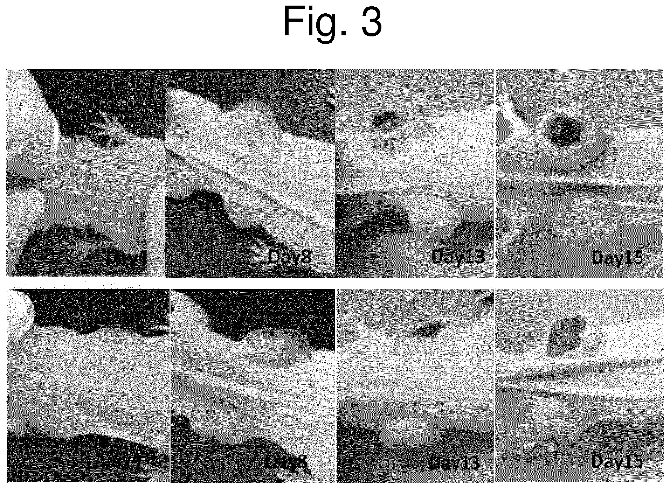

Fig. 3. Injection of allogeneic human myoblasts into tumors of gastrointestinal cancer inflicted nude mice inhibited cancer cell proliferation and reduced the volume of the treated tumors (top) as compared to the controls (bottom).

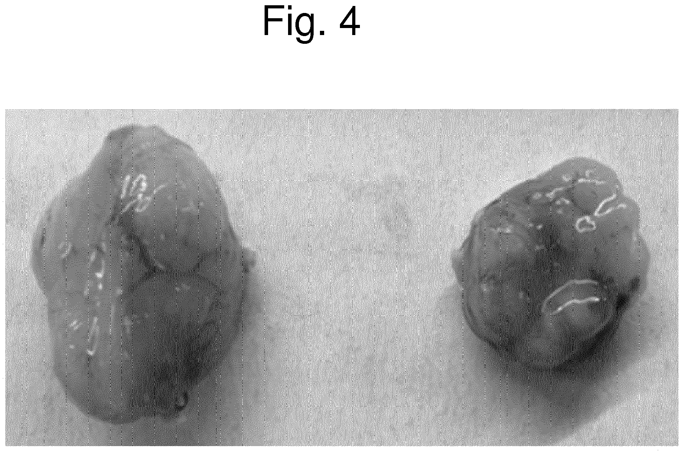

Fig. 4. Administration of the myoblast regime in Study 3 using a total of $12 \times 10^6$ myoblasts reduced the volume of the treated tumor (right) to about 50% of the control (left).

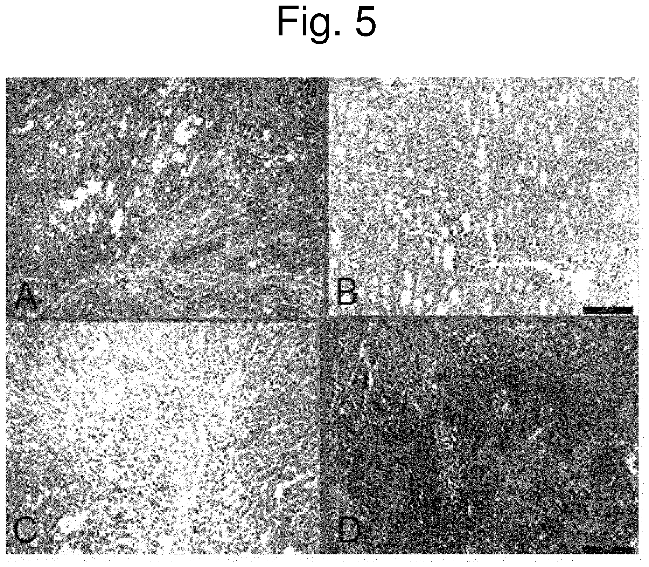

Fig.5. Histologic study of the tumors in Fig. 4 demonstrated (A) presence of large quantity of spindle-shaped myoblasts and myotubes amidst round tumor cells in the myoblast-treated tumor, with abundant cancer cell death appearing as empty space or vacuoles in the tumor sections (B, C). As usual, sections of the control tumor are compact with tumor cells (D). H&E stain. Microscope magnification x100.

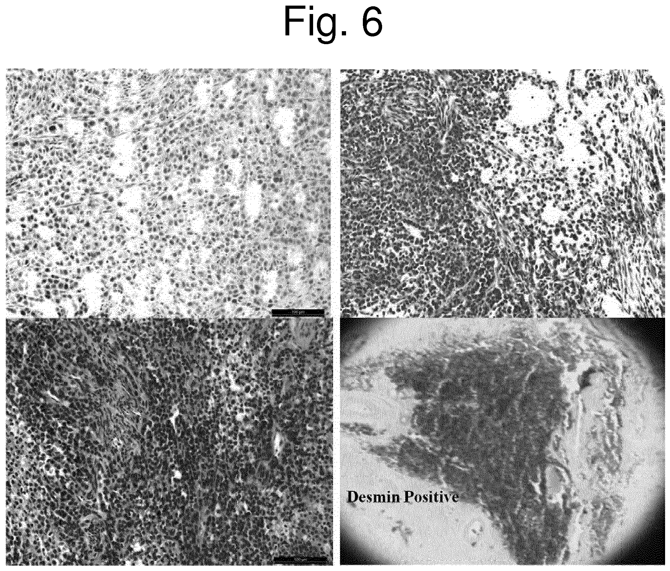

Fig. 6. At higher magnification, histologic study of the tumors in Fig. 4 demonstrated (A) presence of spindle-shaped myoblasts and myotubes amidst round tumor cells in the myoblast-treated tumor, with cancer cell death appearing as empty space or vacuoles in the tumor sections (A, B). (C) Section of the control tumor is compact with cancer cells. Microscope magnification for (A), (B), and (C) is 200x. H&E stain. (D) Myoblasts stained brownish with human desmin immunocytochemistry were observed wrapping around the round cancer cells in sections of the myoblast-treated tumors but not in sections of control tumor. Microscope magnification for (D) is x400.

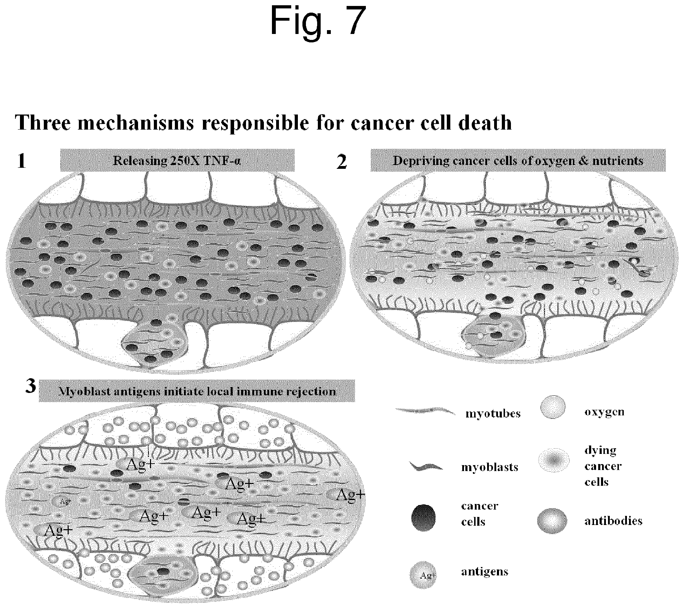

Fig. 7. Initially, three mechanisms were considered to be responsible for inhibition of cancer cell proliferation and cancer cell apoptosis: a) tumor necrosis factor -$\alpha$ (TNF-$\alpha$, TNF-alpha) released following cell membrane breakage during myoblast mitosis and cell fusion killed cancer cells; b) dividing myoblasts and newly developed myotubes competed successfully and had taken away most if not all of the nutrients, L-glutamine and oxygen inside the tumor from the cancer cells; c) injection trauma of allogeneic myoblasts mounted local inflammatory and immunologic attacks on both myoblasts and cancer cells.

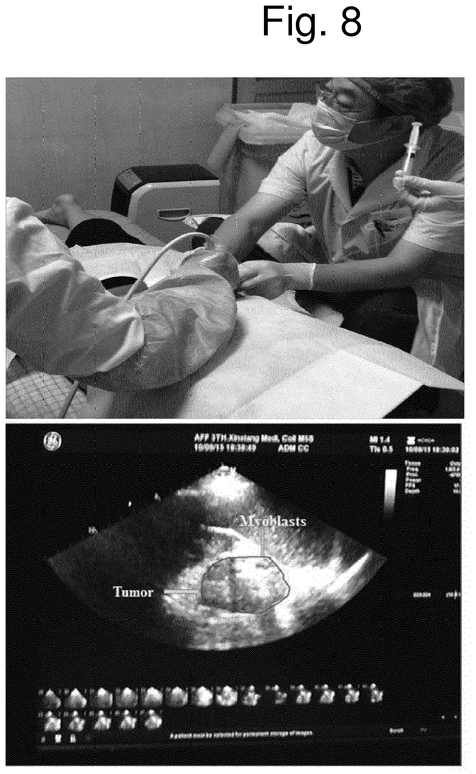

Fig. 8. (A) On September 10, 2015, allogeneic human myoblasts were implanted into an adrenal metastatic small cell carcinoma of the lung with the guidance of a Color Doppler Ultrasound system. (B) About 1 billion allogeneic myoblasts at a concentration of 100 million per ml of patient's own serum were injected into one side (light) of an oblong solid tumor measuring over 52 mm in length. The outline of the adrenal metastatic small cell carcinoma of the lung is traced in dark grey. The other side (dark) of the tumor was not injected and served as a control.

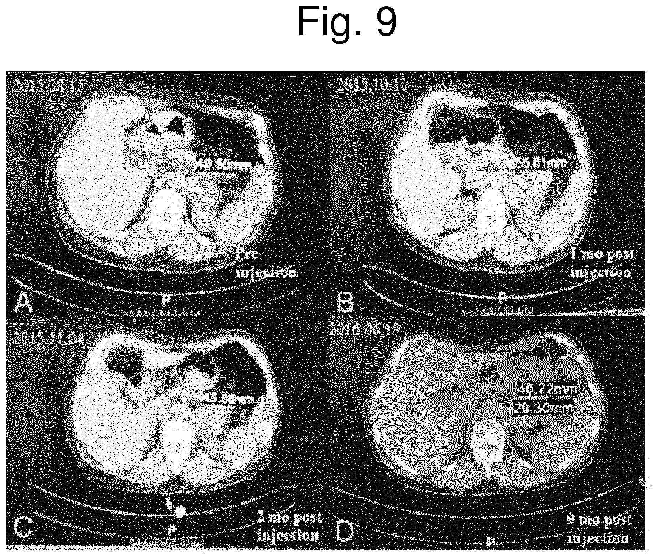

Fig. 9. (A) MRI showed the adrenal metastatic small cell carcinoma of the lung from 49.50 mm in its maximum length at one month before implantation developing to 52.50 mm at the time of myoblast implantation. (B) An increase in tumor size to 55.61 mm in its maximum length was observed at one month after myoblast implantation. (C) At 2 months after myoblast implantation, the tumor size decreased, measuring 45.86 mm in length. A second implantation was administered at 2.5 months after the first. (D) The decrease in tumor size continued until 9 months after the first implantation, with the tumor length being measured at 40.72 mm.

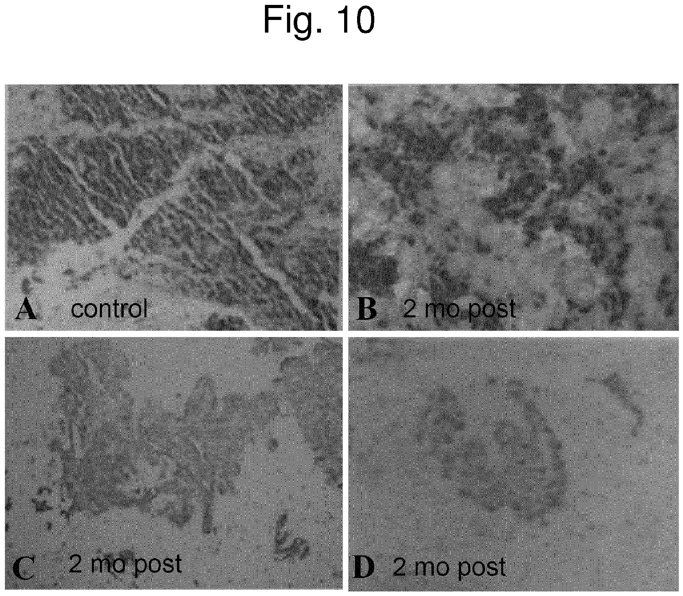

Fig. 10. At 2 months after myoblast implantation, (A) histologic section of the non-injected portion of the adrenal metastatic small cell carcinoma of the lung was densely packed with cancer cells. (B, C, D) Large scale of cancer cell death was apparent in the myoblast-injected portion of the tumor. Microscope magnification x400; H&E stain.

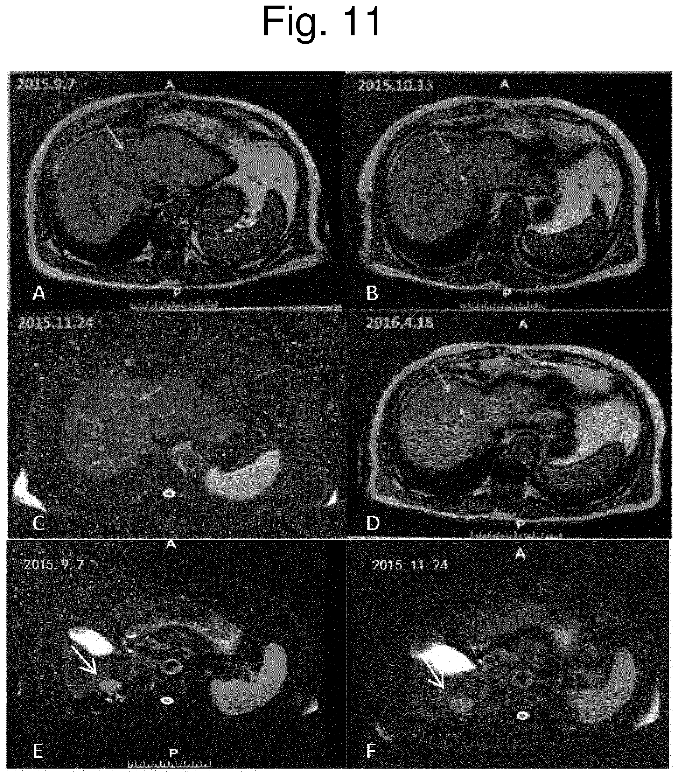

Fig. 11. MRI of liver cancer showing the size and density of Tumor 1 (T1, arrow) before myoblast implantation (A), and at (B) 1 month, (C) 2.5 months, and (D) 7 months after implantation. Whereas (E) the non-injected control Tumor 2 (T2, arrow) in the same liver increased in size in 2 months (F), the myoblast-injected T1 showed a significant decrease in size and density with time, indicating that the myoblasts and myotubes interrupted cancer proliferation and induced cancer apoptosis.

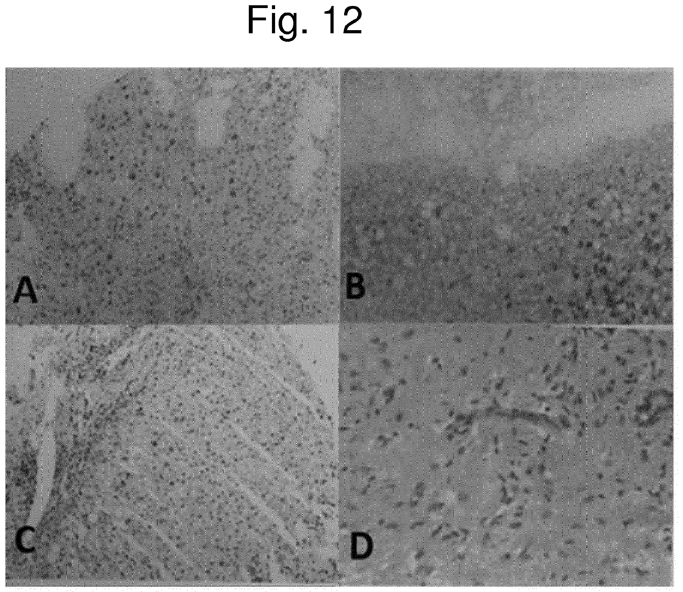

Fig. 12. (A, B) Histologic section of T1 liver tumor demonstrated nodular connective tissue distributed among sclerotic liver tissue 1 month after myoblast implantation. No cancerous tissue was observed. Similar result was obtained for the myoblast-injected T2 tumor at (C) 1.5 months and (D) 9 months after myoblast implantation. Microscope magnification x400; H&E stain.

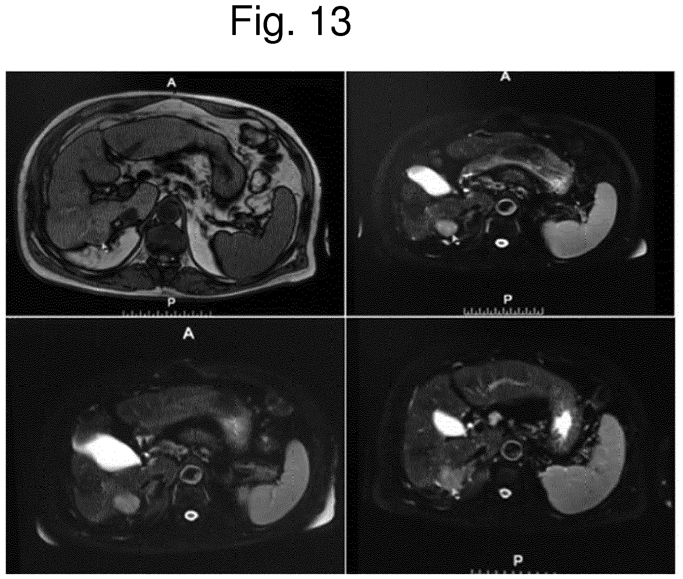

Fig. 13. (A, B, C) MRI showing liver tumor T2 increased in size before myoblast implantation. (D) There was no increase in size at 5 months after myoblast implantation.

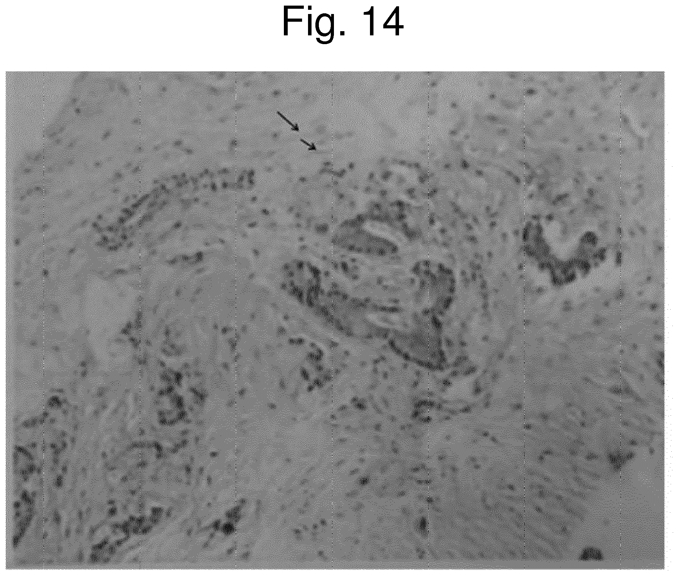

Fig. 14. Reduction in cancer cell number with the presence of myotubes were observed at 1 month after myoblast injection in a histological section of a metastatic adenocarcinoma of the abdominal wall from a 63-year-old man with gastrointestinal cancer. Microscope magnification x400; H&E stain.

## Manufacture of Human Myoblasts

### Muscle Donors

Upon approval of the Institutional Review Board (IRB) of the Cell Therapy Institute and the signing of the Donor Informed Consent, muscle donors were admitted after meeting the Inclusion and Exclusion criteria. They were male volunteers between the ages of 13 and 27. They were certified by a physician as being in good health, not having an AST, ALT, or LD level of more than twice the upper limit of normal, and tested negative for human immunodeficiency virus (HIV), hepatitis B surface antigen (HBSAg), hepatitis C (HCV), syphilis (RPR), and cytomegalovirus (CMV-IgM). They also received the following tests: Chem 24, CBC, and physical examination. Donors will be excluded if they had any chronic or infectious diseases, or were allergic to the local anesthetic.

### Muscle Biopsy

About 2 grams of muscle were removed from the quadriceps muscle using an open biopsy technique under local anesthetic (Lidocaine) in a sterile field of a surgical suite of a hospital. The donor site is sutured and bandaged. No prophylactic antibiotic was used. The donor was discharged after recovery from the surgical procedure to be followed by his physician if infection occurred.

### Preparation of Myoblasts

Biopsy specimen obtained was processed immediately using sterile techniques meeting CFDA approved GMP and ISO 9001 standards. Myoblasts were cultured in growth medium and incubated in 35-37°C and about 7% $CO_2$ as previously described [Law, P.K. et al. Cell Transplantation 1992, 1:235-244; Law, P.K. Methods for Human Myoblast Culture and Transplantation in "Methods in Cell Transplantation", R.G. Landes, Co., Austin, TX 1995]. The myoblasts were frozen at different stages so the time allotted for culturing could be coordinated with a scheduled transplant. The amount of cells frozen and the number of samples were documented. One test vial was reserved for each biopsy when the myoblasts were frozen in liquid nitrogen.

Random samples of the myoblasts were tested for their ability to divide, fuse, and form myotubes [Law, P.K. et al. Cell Transplantation 1992, 1:235-244]. Lot release testing consisted of sterility, endotoxin, mycoplasma, and testing for myoblast identity, purity, potency, viability, and cell count on a pooled sample prior to transplant meeting quality control standards [Law, P.K. et al. Delivery of Biologics for Angiogenesis and Myogenesis in "Practical Handbook of Advanced Interventional Cardiology" ed. by Nguyen, T., Colombo, A., Hu, D., Grines, C. L., and Saito, S. Blackwell Futura, Malden, USA, 3rd edition, 2008, pp. 584-596]. A retain sample of myoblasts was frozen in liquid nitrogen from each transplant.

### Animal Studies

To determine biologic dosing and pharmacokinetics, nude mice were used to test the effect of human myoblasts on the proliferation and the apoptosis of human gastrointestinal cancer cells SGC-7901, and of non-small-cell lung cancer (NSCLC) A549 cells in subcutaneous solid tumors (Table 1).

Table 1. Dosing and pharmacokinetic studies of human myoblasts injected into subcutaneous solid tumors of nude mice established with human non-small-cell lung cancer (NSCLC) A549 cells (Study 1) and with human gastrointestinal (GI) cancer cells SGC-7901 (Studies 2 to 5). Study 6 compared the survival periods of myoblast-treated KM mice previously injected with Ehrlich ascites cells versus control.

| Studies | Cancer | Cell Line | No. of Mice | Myoblasts/ Volume | Days Follow-up |
|---|---|---|---|---|---|
| 1 | NSCLC | A549 | 20 | $2 \times 10^6$/0.2 ml | 92 |
| 2 | GI | SGC-7901 | 8 | $6 \times 10^6$/0.15 ml | 5 |
| 3 | GI | SGC-7901 | 22 | $2 \times 10^6$/0.15 ml | 15 |
| | | | | $10 \times 10^6$/0.15 ml | 4 |
| 4 | GI | SGC-7901 | 5 | $14 \times 10^6$/0.15 ml | 4 |
| | | | | $6 \times 10^6$/0.15 ml | 5 |
| 5 | GI | SGC-7901 | 6 | $28 \times 10^6$/0.15 ml | 9 |
| 6 | EAC | BS344 | 11 | $50 \times 10^6$/0.2 ml | 22 |
| | EAC | BS344 | 10 | 0/0.2 ml | 17 |

In addition, human myoblasts were injected into tumors having Ehrlich ascites cells (BS344 EAC) in KM mice to determine if such intervention might prolong the life-spans of these cancer inflicted mice (Table 1).

Male BALB/c nude mice averaging $17 \pm 1$ g were obtained from Beijing Witung Lihua Limited Co. SCXK (Beijing) 2008 - 0005. Male KM mice averaging $20 \pm 2$g were obtained from Hubei Animal Experimentation Center SCXK (Wuhan) 2014 - 0007. Mice were maintained in compliant with SPF standards. NSCLC and EAC were supplied by the Alfie Inc., Wuhan.

Study 1 involved twenty nude mice, each of which was injected subcutaneously on both sides of the back with 0.2mL of A549 NSCLC cells at a concentration of 25 million/ml (Table 1). They were randomized into two groups (Table 2) after 18 to 20 days when the tumors reached 250-300 mm$^3$ in volume and had developed capillary network of their own (Fig. 1). The control mice then each received injections of 0.2ml of saline into the left tumor, whereas the test mice received 0.2 ml of human myoblasts into the right tumor at a low concentration of 10 million/ml.

The length and width of control and test tumors were measured every week as shown in Figure 2. The volume was calculated using the formula: (length $\times$ width$^2$)/2. Measurements were taken by the same individual at the same positions each time to provide consistency. Mice were sacrificed at 3 weeks with decerebration after being anesthetized and bleed. The tumors were dissected out and weighted. Student's t-tests were used to compare the means of the volumes and weights of myoblast-treated versus control tumors. Tumor inhibitory factor (TIF) for weight was calculated with the formula=(mean control weight - mean test weight) / mean control weight x 100%. Similarly, tumor inhibitory factor (TIF) was calculated for volume with the formula=(mean control volume-mean test volume)/mean control volume x 100%.

Table 2. Effects of human myoblasts on the volume and weight of NSCLC A549 tumors in nude mice (n=10).

| Group | Volume (mm$^3$) | | | | Weight (g) x$\pm$SD (n=8) | TIF (%) |
|---|---|---|---|---|---|---|
| | d0 | d7 | d14 | d20 | | |
| Control | $305.5 \pm 78.6$ | $1032.8 \pm 310.4$ | $1875.3 \pm 546.8$ | $2687.8 \pm 713.4$ | $3.12 \pm 0.88$ | |
| Test | $325.8 \pm 101.4$ | $874.2 \pm 276.5$ | $1586.0 \pm 488.7$ | $2133.7 \pm 638.3^*$ | $2.48 \pm 0.78^*$ | 20.5 |

TIF, tumor inhibitory factor; * indicates p<0.05 by Student's t-test.

Table 2 lists the mean volumes and mean weights of myoblast-treated tumors versus those of controls at day0, day7, day14 and day20. Myoblast-treated tumors were statistically less in weight and volume at d20. The result indicated that myoblast injection not only inhibited tumor growth and volume by 20.6%, but reduced tumor weight by 20.5%, despite the use of only 2 million human myoblasts at a low concentration of 10 million/ml. Studies 2 to 5 consisted of four groups of nude mice aged 4 to 5 week old that had previously received subcutaneous injections of GI SGC-7901 cancer cells on the back and had developed mature tumors of similar sizes on both sides measuring approximately 0.3x0.2x0.2 cm.

Table 1 lists the number of myoblasts, the volume of implantation, and the days of follow-up for each mouse in Studies 2 to 5. These dose-escalation studies were designed to study the pharmacokinetics of myoblasts to determine the safety and efficacy of treating GI cancer using different myoblast concentrations and procedures.

For example, Studies 2 and 5 involved single-time injections of 6 and 28 million myoblasts with follow-up periods of 5 and 9 days respectively. Studies 3 and 4 involved two-time injections of 12 and 20 million myoblasts with follow-up periods of 19 and 9 days, respectively.

In these studies, comparison was made between myoblasts-injected tumors versus control tumors that received similar volume of carrier solution, in terms of tumor size and cancer cell number as revealed by histology of tumor sections using H&E stain.

Figure 3 showed two representative mice from Study 3 each had received 2 million myoblasts in 0.15 ml for the initial 15 days followed by a second injection of 10 million myoblasts in 0.15 ml on day 15. The mice were sacrificed on day 19, and representative myoblast-treated and control tumors were shown in Figure 4. It appeared that injection of myoblasts had inhibited cancer cell proliferation and reduced the volume of the tumor. Administration of the myoblast regime in Study 3 using a total of 12 million myoblasts reduced the volume of the treated tumor to about 50% of the control.

Histologic study at low microscope magnification demonstrated presence of large quantity of spindle-shaped myoblasts and myotubes amidst round tumor cells in the myoblast-treated tumor (Fig. 5A). Abundant cancer cell death appeared as empty space in the tumor sections (Fig. 5B, C). As usual, sections of the control tumor are compact with tumor cells (Fig. 5D).

At higher magnification, histologic study of the tumors in Fig. 4 demonstrated the presence of spindle-shaped myoblasts and myotubes amidst round tumor cells in the myoblast-treated tumor, with cancer cell death appearing as empty space or vacuoles in the tumor sections. The myotubes, failing to become innervated and vascularized in a week, had perished, leaving empty space and vacuoles as observed in Figures 5A, B and C. Myoblasts stained brownish with human desmin immunocytochemistry were observed wrapping around the round cancer cells in sections of the myoblast-treated tumors (Fig. 6), but not in sections of control tumors.

Mechanisms of Cancer Cell Apoptosis

Four mechanisms were considered to be responsible for inhibition of cancer cell proliferation, tumor volume reduction and cancer cell apoptosis:

a) the tumor necrosis factor -$\alpha$ (TNF-$\alpha$) released following cell membrane breakage in the processes of myoblast mitosis and cell fusion killed cancer cells (Fig. 7);
b) dividing myoblasts and newly developed myotubes competed successfully and had taken away most if not all of the nutrients, L-glutamine and oxygen inside the tumor from the cancer cells (Fig. 7);
c) injection trauma of allogeneic myoblasts mounted local inflammatory and immunologic attacks on both myoblasts and cancer cells (Fig. 7).

A fourth mechanism was demonstrated: d) myoblasts wrapping around cancer cells, preventing them from metastasis, and continuing to exert detrimental effects on them (Fig. 6D).

Study 6 : KM mice survival study

Twenty one KM mice were injected intraperitoneal with $2 \times 10^7$ Ehrlich ascites cells each and randomized into test (11 mice) and control (10 mice) groups. One day later, control mice each received intraperitoneal injection of 0.2ml of saline, whereas test mice each received 0.2ml containing 50 million human myoblasts. The number of days of survival of test and control mice were recorded and compared, beginning from the day of cancer cell implantation.

The mean survival period for the control mice was found to be 15.4 $\pm$ 1.5 days which is significantly less than that for the myoblast-injected mice of 18.6 $\pm$ 3.2 days at P< 0.005 by Student's t-test. Life expectancy of the KM mice having Ehrlich ascites cancer was extended by (18.6 - 15.4)/ 18.6 $\times$ 100%= 20.8%.

Results of the Animal Studies

Study 1 demonstrated that injection of allogeneic human myoblasts into solid tumor inhibited mature lung cancer growth, reducing tumor volume and weight by 20.6% and 20.5% respectively, despite the use of low dosage of 2 million human myoblasts at a low concentration of 10 million/ml.

Study 2 to 5 established the safe and effective dose range and optimal pharmacokinetics of the allogeneic human myoblasts in treating mature GI cancer using different myoblast concentrations and procedures. Doses of 2 to 28 million myoblasts administered at 13.3 million/ml to 186.7 million/ml respectively were found to be safe and effective in reducing tumor volume, weight and cancer cell number. The surprising discovery of myoblasts wrapping around the cancer cells in sections of the myoblast-treated tumors (Fig. 6D) suggested a fourth mechanism of cancer cell apoptosis, namely that the myoblasts prevented the cancer cells from metastasis, and continued to exert detrimental effects on them.

Study 6 demonstrated that 50 million allogeneic human myoblasts administered at a high concentration of 250 million/ml could extend the lifespan of mice inflicted with immature Ehrlich ascites cells.

Basis to Initiate Clinical Trial

Results of the cell culture and the animal studies supported our hypothesis that human myoblasts undergoing mitosis and newly-formed myotubes are potent biologics to inhibit cancer cell proliferation, shrinking tumor size and killing cancer cells. Considering that cancer has become the number one killer of human beings, and the demonstrated safety and efficacy of myoblast treatment of patients suffering muscular dystrophy, cardiomyopathy, and Type II diabetes [Law, P. K. Open Journal of Regenerative Medicine 2016; 5: 25-43], benefit versus risk ratio would favor proceeding onto clinical studies.

Clinical Trial

In China, cell transplantation is considered as a medical treatment technology and has been regulated not by the Chinese Food and Drug Administration (CFDA) but by the National Ministry of Health, now called the National Health and Family Planning Commission. As of July 2, 2015 the Commission abolished the necessity to gain approval at the national level for somatic cell transplantation to initiate clinical trials, except for stem cells. Such human studies, however, have to be approved by a Grade 3A hospital that would take on the responsibility of patient safety and register such studies with the Health and Family Planning Commission at the provincial level [National Health and Family Planning Commission of the People's Republic of China Announcement No. 71, 2015-7-2]. The CFDA still has to approve the plant of cell manufacture.

The initial clinical trial using allogeneic human myoblasts to treat cancer patients, its design and protocol thereof, were approved by the Institutional Review Board (IRB) of the Cell Therapy Institute in Wuhan, CHINA. The use of allogeneic human myoblasts as a biologic in clinical studies was also approved by the Third Affiliated Hospital of Xinxiang Medical University in Henan, China.

Cancer patients were admitted after meeting the Inclusion and Exclusion criteria and signing of Patient Informed Consent. They were volunteers between the ages of 55 and 80. They were certified by a physician as being in good health, not having an AST, ALT, or LD level of more than twice the upper limit of normal, and tested negative for human immunodeficiency virus (HIV), hepatitis B surface antigen (HBSAg), hepatitis C (HCV), syphilis (RPR), and cytomegalovirus (CMV-IgM). They also received the following tests: Chem 24, CBC, and physical examination. Subjects will be excluded if they had any infectious diseases.

Being the world's first, this clinical trial proceeded with great caution and care, examining the safety and efficacy of direct implantation of allogeneic human myoblasts into solid tumors of three patients having lung, liver and gastrointestinal cancers respectively.

Case 1

Yang XX, female aged 62, had history of lung cancer metastasized into the brain and the left adrenal gland. Her brain metastasis was treated previously with radiation therapy and chemotherapy for 3 weeks without remission.

The subject underwent allogeneic human myoblast implantation into the adrenal metastatic small cell carcinoma of the lung on September 10, 2015. Implantation was guided with a General Electric (GE) Vivid E9 Color Doppler Ultrasound after piercing with a needle through the abdominal cavity. About 1 billion allogeneic myoblasts at a concentration of 100 million/ml of the patient's own serum were injected into one side of an oblong solid tumor measuring over 52.50 mm in length. The other side of the tumor was not injected and served as a control (Fig. 8).

Some adverse reactions were observed, treated and remised in 10 days. These included temporary reduction in blood pressure down to 82/50 mmHg, coughing, phlegm sputum and headache.

The abdomen was examined with MRI (Siemens, Magnetum-ESSENZA) before and after myoblast implantation, comparing tumor size and density through signals obtained from test and control areas. Examining methods included Axi:IN-PHASE, OPP-PHASE, TSE T2WI/FS, DWI and Cor: TRUFI, T2WI.

MRI showed the tumor from 49.50 mm in its maximum length measured at one month before (Fig. 9A) developing to 52.50 mm at the time of myoblast implantation. An increase in tumor size to 55.61 mm in its maximum length was observed at one month after implantation (Fig. 9B). This could be due to the development of myoblasts into myotubes after cell fusion. However, at 2 months after myoblast implantation, the tumor size decreased, measuring 45.86 mm in length (Fig. 9C).

A second implantation of 1.4 billion allogeneic myoblasts was administered at approximately 120 million/ml on November 26, 2016 with the hope to further interrupt cancer proliferation and to induce cancer apoptosis. The implantation was uneventful and the patient suffered no adverse reaction. The decrease in tumor size continued until 9 months after the first implantation, with the tumor length being measured at 40.72 mm (Fig. 9D).

Pathology of the adrenal tumor biopsies at 2 months postoperatively confirmed the diagnosis of adrenal metastatic small cell carcinoma of the lung, with TIF-1 (+), Vimentin (-), CK (pan) (-), CK7 (-), CK19 (-), SyN (+), CgA (+), Ki-67 (+, 40%) . Histologic examination demonstrated that the non-injected portion of the carcinoma was densely packed with cancer cells (Fig.10A). Large scale of cancer cell death was apparent in the myoblast-injected portion of the tumor (Fig. 10B, C, D).

Case 2

Wang, XX, male, aged 67, previously diagnosed with cardiac malfunction and liver cancer having multiple tumors, underwent allogeneic human myoblast implantation on September 10, 2015. About 700 million myoblasts at a concentration of 100 million/ml of patient's own serum was injected into a solid tumor measuring 35.2 mm x 25.2 mm (Fig. 11A). Implantation was guided with a General Electric (GE) Vivid E9 Color Doppler Ultrasound after piercing with a needle through the upper abdominal cavity and the right anterior wing of the liver where the treated tumor was located. Another tumor in the right posterior wing of the liver, measuring 28.9 mm x 25.4 mm x 26.7 mm, was left untouched to serve as control (Fig. 11E).

The upper abdomen was examined with MRI (Siemens, Magnetum-ESSENZA) at 1 month after myoblast implantation, comparing tumor size and density through signals obtained from test and control areas. Examining methods included Axi:IN-PHASE, OPP-PHASE, TSE T2WI/FS, DWI and Cor: TRUFI, T2WI.

MRI demonstrated that the myoblast-injected Tumor T1 significantly decreased in size and density with time (Fig. 11A to D), whereas the non-injected control Tumor 2 in the same liver increased in size (Fig. 11E, F). This indicated that the implanted myoblasts and developing myotubes interrupted cancer proliferation and induced cancer apoptosis.

At 1 month after myoblast implantation into T1, the myoblast-injected tumor was punctured and two needle biopsies were obtained measuring 1.5 cm and 2.0 cm in length respectively. These were processed for pathologic examination to determine if myoblast implantation caused any cancer cell death. Histology demonstrated nodular connective tissue being distributed among sclerotic liver tissue. No cancerous tissue was observed (Figs. 12A, B), confirming that the implanted myoblasts and developing myotubes interrupted cancer proliferation and induced cancer apoptosis.

On November 26, 2015, the subject received a second myoblast implantation, this time into the previous control tumor T2 positioned at the right posterior wing of the liver (Figs. 13C). About 700 million myoblasts at a concentration of 100 million/ml of patient's own serum were injected into the solid tumor now measuring 33.9 mm x 28.8 mm. It measured 28.9 mm x 25.4 mm x 26.7 mm about 2.5 months ago (Figs. 13A, B). The implantation was uneventful and no adverse reaction was observed.

The outline of the T2 tumor at 5 months after implantation appeared irregular with low density (Fig. 13D). Neither increase nor decrease in size could be ascertained because of its poorly defined outline. Histology of biopsies obtained from this myoblast-injected tumor on January 6 and on August 5, 2016 again did not show any tumor cells (Figs. 12C, D).

Case 3

Shang XX, male, aged 63, had previous been diagnosed with gastric cardia high/middle differentiated ulcer type adenocarcinoma that became metastasized to the abdominal wall and the lymph node posterior to the diaphragm. Surgical removal and chemotherapy did not result in remission of the cancer.

On December 23, 2015, the subject underwent allogeneic human myoblast implantation into the metastasized tumor on the abdominal wall. About 800 million myoblasts at a concentration of 100 million/ml of patient's own serum were injected into the solid tumor. The implantation proceeded without any adverse reaction or untoward after-effect. Histology of the tumor showed a reduction in cancer cell number with the presence of myotubes at 1 month after myoblast injection (Fig. 14).

**Claims**

1. Composition for use in inhibiting growth of a cancer tumor in a host and/or preventing cancer cells from metastasis in a host, wherein the composition is implanted into and around the tumor and/or the cancer cells such that the amount of host serum is reduced upon the implantation and the host serum reduction terminates myoblast proliferation and induces TNF-alpha release and myoblast fusion, wherein the composition comprises a quantum of genetically normal non-host myoblasts and host serum and is adapted to induce myoblast proliferation before being implanted and to induce myoblast fusion upon implantation in that a host serum level in the composition is above a content of 15 % by weight in the composition, and wherein the composition is implanted in at least one dosage containing 75 million to 250 million myoblasts per millilitre of the composition, and wherein the cancer belongs to the group consisting of melanoma cancer, prostate cancer, one or more breast cancers, gastrointestinal cancer, lung cancer, Ehrlich ascites cancer and liver cancer.

2. The composition for use according to claim 1, **characterized in that** the tumor is a solid tumor.

3. The composition for use according to any of claims 1 to 2, **characterized in that** the host is not pre-treated with an immunosuppressant.

4. The composition for use according to any of claims 1 to 3, **characterized in that** the myoblasts are human myoblasts.

5. The composition for use according to any of claims 1 to 4, **characterized in that** the myoblasts were previously isolated, purified and cultured.

6. The composition for use according to any of claims 1 to 5, **characterized in that** the host serum is clotted serum.

7. The composition for use according to any of claims 1 to 6, **characterized in that** the myoblasts are allogeneic.

8. The composition for use according to any of claims 1 to 7, **characterized in that** the composition comprises myoblasts and host serum only.


**Patentansprüche**

1. Zusammensetzung zur Verwendung in der Tumorwachstumshemmung in einem Wirt und/oder der Prävention der Metastasierung bei Krebszellen in einem Wirt, worin die Zusammensetzung in und um den Tumor und/oder die Krebszellen implantiert wird, so dass die Menge an Hostserum bei der Implantierung verringert wird und die Verringerung des Hostserums die Myoblastenverbreitung beendet und die Freigabe von TNF-alpha und die Myoblastenfusion induziert, worin die Zusammensetzung eine Menge an genetisch normalen Nicht-Wirt - Myoblasten umfasst und das Wirtserum adaptiert ist, um vor der Implantierung die Myoblastenverbreitung zu induzieren und bei der Implantierung die Myoblastenfusion zu induzieren, indem ein Wirtserumspiegel in der Zusammensetzung oberhalb eines Gehaltes von 15 Gew.-% in der Zusammensetzung liegt, und worin die Zusammensetzung in mindestens einer Dosierung enthaltend 75 Millionen bis 250 Millionen Myoblasten pro Milliliter der Zusammensetzung implantiert ist, und worin der Krebs zu der Gruppe gehört, die besteht aus Myelomkrebs, Prostatakrebs, einem oder mehr Brustkrebsen, Magen- und Darmkrebs, Ehrlich - Aszites - Krebs und Leberkrebs.

2. Die Zusammensetzung zur Verwendung nach Anspruch 1, charakterisiert dadurch, dass der Tumor ein fester Tumor ist.

3. Die Zusammensetzung zur Verwendung nach irgendeinem der Ansprüche 1 bis 2, charakterisiert dadurch, dass der Wirt nicht mit einem Immunsuppressivum vorbehandelt ist.

4. Die Zusammensetzung zur Verwendung nach irgendeinem der Ansprüche 1 bis 3, charakterisiert dadurch, dass die Myoblasten menschliche Myoblasten sind.

5. Die Zusammensetzung zur Verwendung nach irgendeinem der Ansprüche 1 bis 4, charakterisiert dadurch, dass die Myoblasten zuvor isoliert, gereinigt und kultiviert wurden.

6. Die Zusammensetzung zur Verwendung nach irgendeinem der Ansprüche 1 bis 5, charakterisiert dadurch, dass das Hostserum klumpiges Serum ist.

7. Die Zusammensetzung zur Verwendung nach irgendeinem der Ansprüche 1 bis 6, charakterisiert dadurch, dass die Myoblasten allogen sind.

8. Die Zusammensetzung zur Verwendung nach irgendeinem der Ansprüche 1 bis 7, charakterisiert dadurch, dass die Zusammensetzung nur Myoblasten und Wirtserum umfasst.

**Revendications**

1.  Composition destinée à une utilisation dans l'inhibition de la croissance d'une tumeur cancéreuse dans un hôte et/ou dans la prévention de la formation de métastases de cellules cancéreuses chez un hôte, où la composition est implantée dans et autour de la tumeur et/ou des cellules cancéreuses de telle manière que la quantité de sérum hôte est réduite lors de l'implantation et la réduction de sérum hôte met fin à la prolifération des myoblastes et induit une libération de TNF-alpha et une fusion myoblastique, où la composition comprend une proportion de myoblastes non hôtes génétiquement normaux et de sérum hôte et est appropriée pour induire une prolifération des myoblastes avant d'être implantée et pour induire une fusion myoblastique lors de l'implantation, en ce qu'un niveau de sérum hôte dans la composition est supérieur à une teneur de 15 % en poids dans la composition, et où la composition est implantée dans au moins un dosage contenant 75 millions à 250 millions de myoblastes par millilitre de composition, et où le cancer fait partie du groupe constitué du cancer mélanome, du cancer de la prostate, d'un ou de plusieurs cancers du sein, du cancer gastro-intestinal, du cancer du poumon, du cancer des cellules d'Ehrlich et du cancer du foie.

2.  Composition destinée à une utilisation selon la revendication 1, **caractérisée en ce que** la tumeur est une tumeur solide.

3.  Composition destinée à une utilisation selon l'une quelconque des revendications 1 à 2, **caractérisée en ce que** l'hôte n'est pas prétraité avec un immunosuppresseur.

4.  Composition destinée à une utilisation selon l'une quelconque des revendications 1 à 3, **caractérisée en ce que** les myoblastes sont des myoblastes humains.

5.  Composition destinée à une utilisation selon l'une quelconque des revendications 1 à 4, **caractérisée en ce que** les myoblastes ont été précédemment isolés, purifiés et cultivés.

6.  Composition destinée à une utilisation selon l'une quelconque des revendications 1 à 5, **caractérisée en ce que** le sérum hôte est du sérum coagulé.

7.  Composition destiné à une utilisation selon l'une quelconque des revendications 1 à 6, **caractérisée en ce que** les myoblastes sont allogéniques.

8.  Composition destinée à une utilisation selon l'une quelconque des revendications 1 à 7, **caractérisée en ce que** la composition comprend uniquement des myoblastes et du sérum hôte.

Fig. 1

# Fig. 2

# Fig. 3

# Fig. 4

# Fig. 5

# Fig. 6

# Fig. 7

## Three mechanisms responsible for cancer cell death

# Fig. 8

# Fig. 9

Fig. 10

# Fig. 11

# Fig. 12

# Fig. 13

# Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1407788 A2
- EP 2837683 A1
- WO 9618303 A1

**Non-patent literature cited in the description**

- **ZHE-XI LUO et al.** *Nature,* 2011, vol. 476, 442-445
- **STÖLTING, MNL et al.** *J Urol,* 2013, vol. 189, 1952-1959
- A novel means drug delivery: myoblast-mediated gene therapy and regulatable retroviral vectors. **OZAWA, C.R. et al.** Annual Review of Pharmacology and Toxicology. Annual Review INC, 01 January 2000, vol. 40, 295-317
- **LI, Y.P. ; ROBERT J. SCHWARTZ.** *The FASEB Journal,* 06 April 2001
- **LAW, P.K. et al.** *Cell Transplantation,* 1992, vol. 1, 235-244
- Methods for Human Myoblast Culture and Transplantation. **LAW, P.K.** Methods in Cell Transplantation. R.G. Landes, Co, 1995
- Delivery of Biologics for Angiogenesis and Myogenesis. **LAW, P.K. et al.** Practical Handbook of Advanced Interventional Cardiology. 2008, 584-596
- **LAW, P. K.** *Open Journal of Regenerative Medicine,* 2016, vol. 5, 25-43
- *National Health and Family Planning Commission of the People's Republic of China Announcement No. 71,* 02 July 2015